# EUROPÄISCHE PATENTSCHRIFT

(11) **EP 0 336 184 B1**
(45) Veröffentlichungstag und Bekanntmachung des Hinweises auf die Patenterteilung: **13.01.1993**
(21) Anmeldenummer: 89104900.9
(22) Anmeldetag: 18.03.1989
(51) Int. Cl.: C07C 233/34, C08G 18/38, C08G 18/10

(54) **Monoformylierte 3,3'-Diaminodipropylamine, ein Verfahren zu deren Herstellung und deren Verwendung**
Monoformylated 3,3'-diaminopropyl amine, process for its preparation and its use
3,3'-diaminodipropylamine monoformylée, procédé pour sa préparation et son utilisation

(30) Priorität: 02.04.1988 DE 3811342
(43) Veröffentlichungstag der Anmeldung: 11.10.1989
(73) Patentinhaber: BAYER AG, 51368 Leverkusen (DE)
(72) Erfinder: Scholl, Hans-Joachim, Dr., D-5000 Köln 80 (DE); Reiff, Helmut, Dr., D-5090 Leverkusen 1 (DE)

(56) Entgegenhaltungen:
- EP-A- 0 205 131
- DE-A- 2 523 633
- DE-C- 735 771
- US-A- 4 238 409

## Beschreibung

Die vorliegende Erfindung betrifft neue, monoformylierte 3,3'-Diaminodipropylamine bzw. Gemische, ein Verfahren zu deren Herstellung aus Acrylnitril/Formamid und nachfolgender Hydrierung und deren Verwendung als Kettenverlängerungsmittel bei der Herstellung von Polyurethanen nach dem Isocyanat-Polyadditionsverfahren.

Es wurden die neuen, monoformylierten 3,3'-Diamodipropylamin-Gemische der Formel I und II gefunden 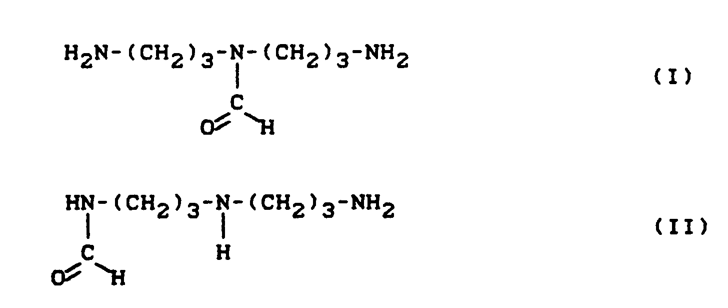
Es wurde weiterhin ein Verfahren zu ihrer Herstellung gefunden, das dadurch gekennzeichnet ist, daß man Acrylnitril und Formamid unter Zusatz eines 4-Aminopyridin-Derivates zum N,N-Bis-(2-cyanethyl)-formamid umsetzt und nachfolgend zum monoformylierten 3,3'-Diaminopropylamin-Gemisch der Formel I und II hydriert.

Umsetzungen von Acrylnitril und Formamid in Gegenwart von Basen sind beschrieben worden:
So erhält man nach DRP 735771 N,N-Bis-(2-cyanethyl)-formamid aus 2 Mol Acrylnitril und 1 Mol Formamid mit katalystischen Mengen Natrium.

Nach den offenbarten, drastischen Herstellungsmethoden sind merkliche Anteile an Verunreinigungen zu erwarten, z.B. weitere Umsetzungen von N,N-Bis(2-cyanethyl)-formamid mit Acrylnitril, deren Vorhandensein zur Reindarstellung der erfindungsgemäßen Diamingemische I und II prohibitiv ist.

Umsetzungen von Acrylnitril mit Formamid in Gegenwart einer tertiären Aminbase zu 2-(N-Formylamino)-propionitril beschreibt die DE-OS 3 520 982. Selektivität und Ausbeuten sind nach den dort gemachten Angaben hoch. Das Bisaddukt N,N-Bis-(2-cyanethyl)-formamid wurde allerdings nicht gefunden.

Es muß daher als äußerst überraschend angesehen werden, daß nach dem erfindungsgemäßen Verfahren N,N-Bis-(2-cyanethyl)-formamid überhaupt entsteht, zumal in hohen Ausbeuten, guter Selektivität und Reinheit.

Bezogen auf 1 Mol Acrylnitril können beispielsweise 0,5 bis 10 Mol Formamid eingesetzt werden. Vorzugsweise beträgt die Menge 0,5 bis 2 Mol.

Als 4-Aminopyridin-Derivate kommen beispielsweise solche der Formel (III) in Frage 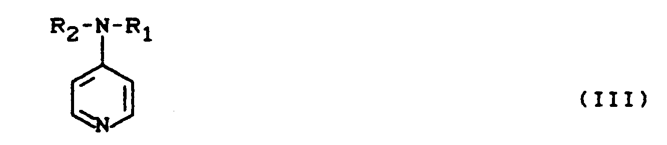
in der
R₁ und R₂ unabhängig voneinander für einen einbindigen C₁- bis C₆-Alkylrest oder R₁ und R₂ gemeinsam für einen zweibindigen C₃- bis C₅-Alkylrest stehen.

Bevorzugt wird N,N-Dimethyl-4-aminopyridin oder 4-Pyrrolidinopyridin eingesetzt.

Die Einsatzmenge dieser katalytisch wirkenden Base ist nicht kritisch. Zur Erzielung eines hinreichend schnellen Umsatzes werden üblicherweise 0,5 bis 10 Mol-% pro Mol Acrylnitril verwendet.

Acrylnitril kommt als technische Ware zum Einsatz. Diese Ware enthält in geringer Menge übliche Polymerisationsinhibitoren.

Das Verfahren kann lösungsmittelfrei oder vorzugsweise unter Mitverwendung eines Lösungsmittels durchgeführt werden.

Zur Erhöhung der Selektivität ist Lösungsmittelzusatz bei steigenden Reaktionstemperaturen zunehmend erforderlich. Hierfür eignen sich polare Lösungsmittel wie z.B. Tetrahydrofuran, Dimethylacetamid oder Acetonitril. Acetonitril ist besonders bevorzugt.

Geeignete Reaktionstemperaturen sind beispielsweise solche im Bereich von 0 bis 120°C, vorzugsweise im Bereich von 20 bis 100°C, wobei insbesondere die lösungsmittelfreie Variante vorzugsweise bei Raumtemperatur durchgeführt wird.

Das Arbeiten bei Normaldruck ist bevorzugt, jedoch kann auch unter erhöhten Druck gearbeitet werden.

Im übrigen erfordert das erfindungsgemäße Verfahren keine verfahrenstechnischen Besonderheiten. Das gleiche gilt für die Aufarbeitung. Nach Abtrennung der Niedrigsieder, Formamid und des Katalysators (z.B. durch Destillation oder Dünnschichten) fällt N,N-Bis-(2-cyanethyl)-formamid als schwach gefärbte Flüssigkeit an, die von üblicher Zwischenproduktreinheit ist, so daß auf weitere Reinigungsprozeduren verzichtet werden kann, weil prohibitive Verunreinigungen des Standes der Technik nach dieser erfindungsgemäßen Verfahrensweise nicht entstehen.

Die folgende Stufe des erfindungsgemäßen Verfahrens besteht in der an sich bekannten Hydrierung der beiden Nitrilgruppen des N,N-Bis-(2-cyanethyl)-formamids; beispielsweise in Methanol/Ammoniak als Lösungsmittel unter Verwendung von Raney-Nickel-Eisen als Hydrierkatalysator bei Temperaturen von etwa 20 bis 60°C und einem Wasserstoffdruck von etwa 20 bis 50 bar. Nach Abtrennung des Katalysators und der Niedrigsiederanteile: Ammoniak/Methanol sind direkt in ausgezeichneter Qualität die erfindungsgemäßen monoformylierten 3,3'-Diaminopropylamin-Gemische der Formel I und II erhältlich.

Der erfindungswesentliche Punkt ist darin zu sehen, daß erst aufgrund des erfindungsgemäßen Verfahrens zur Herstellung von N,N-Bis(2-cyanethyl)-formamid ein genügend reines Ausgangsprodukt zur Verfügung steht, das die Herstellung der erfindungsgemäßen monoformylierten Diamine I und II in einfacher Weise und guter Qualität nach an sich bekannten Hydriertechniken ermöglicht.

Das erfindungsgemäße monoformylierte 3,3'-Diaminodipropylamin-Gemisch ist eine bei Raumtemperatur dünnflüssige Substanz, die keinerlei Kristallisationsneigung erkennen läßt. Der Geruch ist nur schwach aminisch.

Das erfindungsgemäße, monoformylierte 3,3'-Diaminodipropylamin-Gemisch stellt ein neuartiges, spezielles Kettenverlängerungsmittel für die Herstellung von Polyurethankunststoffen nach dem Isocyanat-Polyadditionsverfahren dar, in dem zunächst eine Kettenverlängerung über die beiden Amingruppen vorgenommen werden kann und nachfolgend gegebenenfalls weitere Umsetzungen der 〉N-CHO-Funktion möglich sind.

Mögliche Umsetzungen sind Oxidation, Reduktion oder hydrolyt- und/oder thermische Spaltung.

Das erfindungsgemäße Diamingemisch kann beispielsweise zu Aufbau von gelösten Polyurethanpolyharnstoffen nach dem Lösungspolyadditionsverfahren verwendet werden. Dazu wird ein NCO-Prepolymer mit einem NCO-Gehalt von 0,5 - 10 Gew.-% in einem Lösungsmittel gelöst und schrittweise mit dem erfindungsgemäßen Diamingemisch verlängert bis die gewünschte Viskosität von ca. 5000 bis ca. 70 000 mPas erreicht wird. Der Feststoffgehalt beträgt üblicherweise 20 - 40 Gew.-%. Man kann auch vorteilhaft so verfahren, daß das erfindungsgemäße Diamingemisch vorgelegt wird und die Polyaddition durch Zugabe der NCO-Prepolymerlösung durchgeführt wird.

Die zu verwendenden Lösungsmittel gehören zum Stand der Technik. Es sind beispielsweise Dimethylformamid, Dimethylacetamid, N-Methylpyrrolidon aber auch Aromaten/Alkohol-Gemische wie etwa Toluol-Isopropanol.

Die Endeigenschaften der Polyurethanharnstoffe lassen sich gezielt einstellen, indem man beispielsweise neben dem erfindungsgemäßen Diamingemisch zusätzlich gezielt Monoamine (partieller Kettenabbruch, Viskositätsverminderung), in der PUR-Chemie übliche Diamine, gegebenenfalls mit Salzgruppen (Carboxyl- und oder Sulfonatgruppen) oder aber Triamine (Verzweigung, Viskositätssteigerung, Vernetzung) einsetzt.

Das erfindungsgemäße Diamingemisch kann weiterhin anstelle üblicher Diamine besonders vorteilhaft zur Herstellung wäßr. Polyurethandispersionen verwendet werden wie sie beispielsweise in Angew. Makromol. Chemie 26, 1972, 85 - 106 beschrieben sind. Auch in diesem Fall kann gegebenenfalls eine zusätzliche Reaktion an der 〉N-CHO-Gruppe durchgeführt werden.

Auch als aminischer Härter für Epoxide kann das erfindungsgemäße Diamingemisch vorteilhaft verwendet werden. So kann beispielsweise in gängigen 2-Komponenten-Epoxid-Klebern der Härter gegen das erfindungsgemäße Diamingemisch ausgetauscht werden, wobei farblose, elastifizierte und sehr gut haftende Klebstoffe gebildet werden.

Die vorliegende Erfindung soll anhand der folgenden Beispiele näher erläutert werden (%-Angaben beziehen sich auf Gew.-%).

### Beispiele

### Beispiel 1 (Herstellung von N,N'-Bis-(2-cyanethylformamid)

In eine Lösung aus 212 g Acrylnitril, 212 g Acetonitril und 180 g Formamid werden bei Raumtemperatur unter Rühren 14,7 g N,N-Dimethyl-4-aminopyridin eingetragen. Die klare Lösung wird 21 Stunden unter Rückflußbedingungen erhitzt, wobei die Innentemperatur von 86°C auf ca. 92°C anstieg.

Die Reaktionslösung wurde nachfolgend von Niedrigsiedern befreit (30 - 40°C/20 bis 1 mbar) und die hellgelbe, klare Rohlösung unter Dünnschichtbedingungen (145°C 0,1 mbar) von restlichen, niedriger siedenden Anteilen abgetrennt. Die so erhaltende hellgelbe, klare Flüssigkeit besteht zu 99 % (GC) aus N,N'-Bis-(2-cyanethyl)-formamid.
- Ausbeute:: 170 g, Umsatz: 59 % (bezogen auf Acrylnitril)
- Selektivität:: 95%

| Analyse (%): | C | H | N |
|---|---|---|---|
| gefunden: | 55,3 | 6,3 | 27,8 |
| Theorie: | 55,6 | 6,0 | 27,8 |
| (bezogen auf C₇H₉N₃O) | | | |

### Beispiel 2 (Herstellung von N,N'-Bis-(2-cyanethyl)-formamid

In eine Lösung aus 770 g Acrylnitril und 433 g Formamid werden bei Raumtemperatur unter Rühren 27,5 g N,N′-Dimethyl-4-aminopyridin eingetragen. Man läßt die klare Lösung 4 Tage bei Raumtemperatur stehen, befreit die Reaktionslösung von Niedrigsiedern (Raumtemperautr/20 bis 1 mbar) und trennt restliche Siedeanteile unter Dünnschichtbedingungen (145°C/0,1 mbar) ab. Die so erhaltene Sumpf-Flüssigkeit ist praktisch farblos und klar.
- Ausbeute:: 621 g N,N'-Bis-(2-cyanethyl)-formamid
- Reinheit:: 98 % (GC)
- Umsatz:: 53 % (bezogen auf Acrylnitril
- Selektivität:: 96 %

### Beispiel 3 (Herstellung monoformylierter 3,3'-Diaminodipropylamin-Gemische der Formel I und II

In einem Rührautoklav werden 255 g N,N'-Bis-(2-cyanethyl)-formamid aus Beispiel 2 mit 1000 ml Methanol, 500 ml Ammoniak und 40 g Raney-Nickel-Eisen (Ni/Fe Verhältnis: 85/15 versetzt. Bei 30°C und 30 bis 50 bar Wasserstoffdruck wird bis zum Ende der Wasserstoffaufnahme gerührt. Anschließend wird entspannt, vom Katalysator abfiltriert und der Niedrigsiederanteil: Ammoniak und Methanol abgetrennt.

Man erhält 262 g (98 % Ausbeute) fast farbloses, klares, dünnflüssiges Amingemisch der Formel I und II, das nach 1 H-NMR-Untersuchungen im molaren Verhältnis von ca. 50:50 % vorliegt.

| Analyse (%): | C | H | N |
|---|---|---|---|
| gefunden | 53,0 | 10,5 | 26,4 |
| Theorie | 52,8 | 10,7 | 26,4 |
| (bezogen auf C₇H₁₇N₃O) Der Basen-N betrug 17,4 % (Theorie 17,6 %) | | | |

### Beispiel 4 (Vergleichsbeispiel zu Beispiel 3)

®Luprintan PFD (Fa. BASF), ein N,N'-Bis-(2-cyanethyl)-formamid nach dem bisherigen Stand der Technik wird nachfolgend vergleichweise untersucht:

Entsprechend Beispiel 3 werden 255 g Luprintan PFD hydriert. Man erhält 237 g (88 % Ausbeute) eines dunkel gefärbten dickflüssigen Amingemisches mit einem Basen-N von 15,2 % (Theorie 17,6 %).

| Analyse (%) | C | H | N |
|---|---|---|---|
| gefunden | 51,8 | 9,9 | 25,6 |
| Theorie | 52,8 | 10,7 | 26,4 |
| (bezogen auf C₇H₁₇N₃O) | | | |

Die vorliegenden Daten weisen dieses Produkt von vergleichsweise minderer Qualität aus.

Die nachfolgenden Beispiele behandeln Verwendungsmöglichkeiten der erfindungsgemäßen monoformylierten Amingemische (I) und (II):

### NCO-Prepolymer 1:

830,5 g (0,489 mol) Polyester aus Adipinsäure, 1,6-Hexandiol und Neopentylglykol vom Molekulargewicht 1700 und 28,3 (0,013 mol) eines monofunktionellen auf n-Butanol gestartetem Ethylenoxid/Propylenoxidpolyethers vom Molgewicht 2150 werden bei 110°C 30 min im Vakuum entwässert. Nach Abkühlen auf 55 - 60°C wird eine Mischung aus 122 g (0,55 mol) 1-Isocyanatomethyl-5-isocyanato-1,3,3-trimethylcyclohexan und 92,5 g (0,55 mol) 1,6-Diisocyanatohexan zugegeben. Nach 15 Minuten wird auf 120°C erwärmt und 2 h bei dieser Temperatur nachgerührt. Nun wird mit 340 ml Aceton zu einem Feststoffgehalt von 80 % gelöst. Der NCO-Gehalt der Lösung beträgt 3,3 % (Theorie 3,7 %).

### NCO-Prepolymer 2:

403,7 g (0,238 mol) eines Polyesters aus Adipinsäure, 1,6-Hexandiol und Neopentylglykol vom Molgewicht 1700 werden bei 110°C 30 min im Wasserstrahvakuum entwässert. Nach Abkühlen auf 60 - 70°C setzt man 96,3 g (0,434 mol) 1-Isocyanatomethyl-5-isocyanato-1,3,3-trimethylcyclohexan zu und erwärmt nach ca. 15 min auf 110°C. Nach 1,5 h löst man 70 %ig in Toluol ein. Der NCO-Gehalt der Lösung beträgt 1,8 % (Theorie 2,3 %).

### Verwendungsbeispiele

### A Polyurethanharnstoffe durch Lösungspolyaddition

A 1) Zu einer Lösung aus 3,81 g (23,7 mmol) des erfindungsgemäßen Diamingemisches aus Beispiel 3 und 1,6 (5,4 mmol) Oktadecylisocyanat in einer Mischung aus 80 g Toluol, 80 g Isopropanol und 32 g Ethylenglykolmonoethylether tropft man portionsweise langsam 100 g Prepolymerlösung 2. Man erhält eine blaß-gelbe, klare Lösung mit einem Feststoff von 25,5 % und einer Viskosität (23°C) von 7500 mPas.
   Glasplatten und Aluminiumfolien werden mit der Lösung beschichtet und das Lösungsmittel bei 130°C verdampft. man erhält eine optisch klare, weiche aber klebfreie Beschichtung. Der Polyurethanharnstoffilm ist sehr elastisch und zeigt auf Aluminium eine ausgezeichnete Haftung.
A 2) Zu einer Lösung von 3,81 g (0,024 mol) des erfindungsgemäßen Amingemisches gemäß Beispiel 3 in 192 g N,N-Dimethylformamid tropft man bei 50°C eine Mischung aus 100 g Prepolymerlösung 2 und 1,8 g Octadecylisocyanat innerhalb 2 Stunden zu. Nach Abkühlen auf Raumtemperatur erhält man eine leicht gelbe, klare Lösung mit einem Feststoffgehalt von 25 % und einer Viskosität von 11.000 mPas. Der bei 130°C erzeugte Film ist farblos und klar.
A 3) Man arbeitet völlig analog A 2, setzt jedoch das Amingemisch gemäß Beispiel 4 ein. Die erhaltene Polymerlösung ist braun und leicht opak, die Viskosität beträgt lediglich 1000 mPas. Die entsprechenden Filmeigenschaften sind ähnlich.

### B Herstellung von Polyurethanharnstoffdispersionen in Wasser

B 1) 250 g Prepolymerlösung 1 werden mit 450 ml Aceton auf 33 % Feststoffgehalt verdünnt und bei 50 - 55°C mit einer Mischung aus 4,15 g (0,011 mol) 50 %iger wäßriger Lösung des 2-Aminoethyl-β-aminoethansulfonsäure-natriumsalzes, 0,71 g (0,014 mol) Hydrazinhydrat und 6,8 g (0,04 mol) des erfindungsgemäßen Amingemisches nach Beispiel 3 in 50 ml entsalztem Wasser, versetzt. Nach 15 Minuten wird mit 440 ml entsalztem Wasser in 6 min dispergiert. Nach dem Abdestillieren des Acetone im Wasserstrahlvakuum erhält man eine feinteilige Dispersion mit einem Feststoffgehalt von 33,8 %. Der bei 120°C erzeugte Film ist optisch klar und farblos.
B 2) Man verfährt völlig analog Beispiel A, setzt jedoch anstelle des gemäß Beispiel 3 hergestellten Amins nunmehr 6.8 g (0,04 mol) Amin aus Vergleichsbeispiel 4 ein. Die aus dem Versuch resultierende Dispersion ist ebenfalls feinteilig, es setzt sich jedoch über Nacht wenig dunkelbraunes Sediment ab.
B 3) Als Vergleichsbeispiel zu Beispiel A und B anstelle der beiden erfindungsgemäßen Amine 7,22 g (0,043 mol) 1-Aminomethyl-5-amino-1,3,3-trimethylcyclohexan (Isophorondiamin eingesetzt. Man erhält eine grobteilige Dispersion mit einem Feststoffgehalt von 34 %. Der bei 120°C erzeugte Film zeigt ein opakes Aussehen, was auf Inhomogenitäten hinweist.

Für die Dispersionen B 1 - B 3 werden folgende Daten bestimmt:

| | B 1 | B 2 | B 3 |
|---|---|---|---|
| Feststoffgehalt | 33,8 % | 34,2 % | 34,0 % |
| pH-Wert | 7 - 8 | 7 - 8 | 7 - 8 |
| Sulfongehalt | 0,42 % | 0,42 % | 0,42 % |
| Teilchengröße (nm) | 95 | 240 | 220,800 |
| Verteilung | eng, monomodal | wenig braunes Sediment | bimodal |

### C) Verwendung des erfindungsgemäßen Diamingemisches als Härter für Epoxidharze

1 g eines käuflichen Epoxidharzes (Uhu 300 Binder, Fa. Uhu, D 7580, Bühl) wird mit 0,34 g des erfindungsgemäßen Diamingemisches gemäß Beispiel 3 innig vermischt. Das Produkt ist nach 10 Minuten bei 100°C ausgehärtet. Der Film aus dem 2-Komponentenkleber ist kratzfest, Farblos und optisch klar.

Wiederholt man den Versuch mit 0,35 g des Diamingemisches gemäß Vgl.-Beispiel 4, so erhält man bei sonst völlig analogem Befund einen gelb-braunen statt völlig farblosen Film.

Die Aushärtung ist auch über Nacht bei Raumtemperatur möglich. Die Ergebnisse sind identisch.

## Patentansprüche

1. Monoformylierte 3,3'-Diaminodipropylamin-Gemische der Formel I und II im Verhältnis 1:99 bis 99:1, vorzugsweise 20:80 bis 80:20 Mol-%

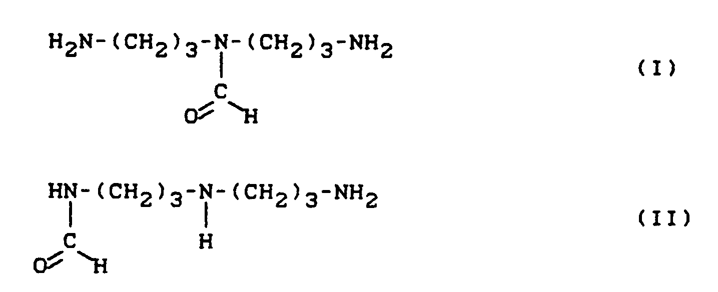

2. Verfahren zur Herstellung von monoformylierten 3,3'-Diaminodipropylamin-Gemischen nach Anspruch 1, dadurch gekennzeichnet, daß man Acrylnitril und Formamid unter Zusatz eines 4-Aminopyridin-Derivates umsetzt und das so erhältliche N,N'-Bis-(2-cyanethyl)-formamid anschließend hydriert.

3. Verfahren nach Anspruch 2, dadurch gekennzeichnet, daß man ein 4-Aminopyridin-Derivat der Formel 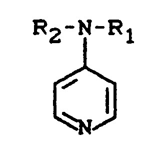 einsetzt, in der
R₁ und R₂ unabhängig voneinander für einen einbindigen C₁- bis C₆-Alkylrest oder R₁ und R₂ gemeinsam für einen zweibindigen C₃- bis C₅-Alkylrest stehen.

4. Verfahren nach einem der Ansprüche 2 oder 3, dadurch gekennzeichnet, daß bezogen auf Acrylnitril 0,5 bis 10 Mol-% eines 4-Aminopyridin-Derivates zum Einsatz kommen.

5. Verwendung von monoformylierten 3,3'-Diaminodipropylamin-Gemischen nach Anspruch 1 als alleiniges oder anteiliges Kettenverlängerungsmittel bei der Herstellung von Polyurethankunststoffen nach dem Isocyanat-Polyadditionsverfahren.

## Claims

1. Monoformylated 3,3'-diaminodipropyl amine mixtures corresponding to formulae I and II in a ratio of 1:99 to 99:1 and preferably 20:80 to 80:20 mol-%:

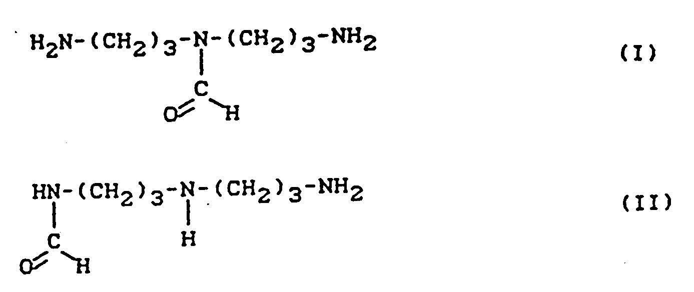

2. A process for the production of the monoformylated 3,3'-diaminodipropyl amine mixtures claimed in claim 1, characterized in that acrylonitrile and formamide are reacted in the presence of a 4-aminopyridine derivative and the N,N'-bis-(2-cyanoethyl)-formamide obtained is subsequently hydrogenated.

3. A process as claimed in claim 2, characterized in that the 4-aminopyridine derivative used corresponds to the following formula 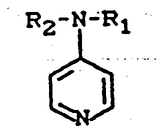 in which
R₁ and R₂ independently of one another represent a single-bonded C₁₋₆ alkyl radical or R₁ and R₂ together represent a double-bonded C₃₋₅ alkyl radical.

4. A process as claimed in claim 2 or 3, characterized in that the 4-aminopyridine derivative is used in a quantity of 0.5 to 10 mol-%, based on acrylonitrile.

5. The use of the monoformylated 3,3'-diaminodipropyl amine mixtures claimed in claim 1 as sole chain extender or co-chain extender in the production of polyurethane plastics by the isocyanate polyaddition process.

## Revendications

1. Mélanges de N,N-bis-(3-aminopropyl)-amines monoformylées de formules I et II dans un rapport molaire de 1:99 à 99:1, de préférence 20:80 à 80:20 :

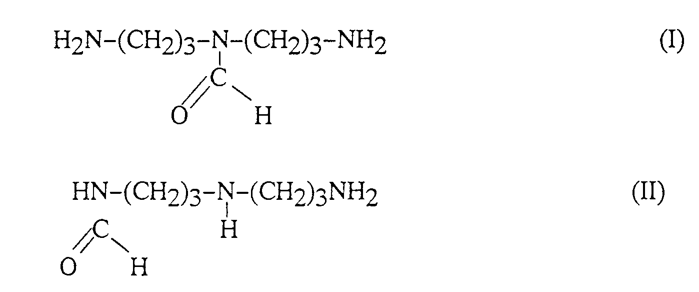

2. Procédé pour la fabrication de mélanges de N,N-bis-(3-aminopropyl)-amines monoformylées selon la revendication 1, caractérisé en ce que l'on fait réagir l'acrylonitrile et le formamide avec addition d'un dérivé de 4-aminopyridine et l'on hydrogène ensuite le N,N-bis-(2-cyanoéthyl)-formamide.

3. Procédé selon la revendication 2, caractérisé en ce que l'on utilise un dérivé de 4-aminopyridine de formule : 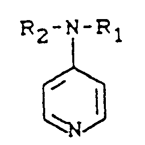 dans laquelle
R₁ et R₂ représentent indépendamment l'un de l'autre un reste alkyle à 1 liaison en C₁-C₆ ou bien R₁ et R₂ pris ensemble représentent un reste alkyle à 2 liaisons en C₃-C₅.

4. Procédé selon la revendication 2 ou 3, caractérisé en ce que l'on utilise 0,5 à 10 mol% d'un dérivé de 4-aminopyridine par rapport à l'acrylonitrile.

5. Utilisation des mélanges de N,N-bis-(3-aminopropyl)-amines monoformylées selon la revendication 1 comme agent d'allongement de chaînes unique ou partiel dans la fabrication de matière plastique de polyuréthanne selon le procédé de polyaddition des isocyanates.
